# FASCICULE DE BREVET EUROPEEN

(11) **EP 1 805 128 B1**
(45) Date de publication et mention de la délivrance du brevet: **09.07.2008**
(21) Numéro de dépôt: 05809097.8
(22) Date de dépôt: 18.10.2005
(51) Int. Cl.: C07C 57/075

(54) **PROCEDE AMELIORE DE FABRICATION D'ANHYDRIDE (METH)ACRYLIQUE**
VERBESSERTES VERFAHREN ZUR HERSTELLUNG EINES (METH)ACRYLANHYDRIDS
IMPROVED METHOD FOR THE PRODUCTION OF (METH)ACRYLIC ANHYDRIDE

(30) Priorité: 26.10.2004 FR 0411396
(43) Date de publication de la demande: 11.07.2007
(73) Titulaire: ARKEMA FRANCE, 92700 Colombes (FR)
(72) Inventeur: PAUL, Jean-Michel, F-57070 METZ (FR)
(74) Mandataire: Bonnel, Claudine
(86) Numéro de dépôt international: PCT/FR2005/002573
(87) Numéro de publication internationale: WO 2006/045919

(56) Documents cités:
- EP-A- 1 319 650
- FR-A- 2 592 040
- GB-A- 2 285 983
- US-A1- 2002 161 260

## Description

La présente invention concerne un procédé amélioré de fabrication d'anhydride (méth)acrylique par transanhydrification entre l'acide (méth)acrylique et l'anhydride acétique.

Par anhydride (méth)acrylique que l'on désignera par la suite par A(M)A₂O, on entend l'anhydride méthacrylique AMA₂O ou l'anhydride acrylique AA₂O.

La synthèse de l'anhydride (méth)acrylique par transanhydrification entre l'acide (méth)acrylique et l'anhydride acétique a fait l'objet de nombreux brevets. On peut citer plus particulièrement la demande FR 2 592 040 qui décrit un tel procédé en l'absence de catalyseur et en présence d'un inhibiteur de polymérisation. La mise en oeuvre de ce procédé se heurte cependant à des problèmes de polymérisation, l'une des principales difficultés résidant dans le choix des inhibiteurs de polymérisation. Il est en effet bien connu que l'un des points délicats de la fabrication et/ou de la purification de monomères (méth)acryliques provient du fait que ces composés sont instables et ont tendance à évoluer facilement vers la formation de polymères. Cette évolution, causée par une réaction radicalaire due à l'effet de la température, est particulièrement favorisée lors des étapes de synthèse et de purification de ces monomères, par exemple dans les étapes de distillation. Il en résulte la formation, dans les équipements des installations, de dépôts de polymères solides qui finissent par provoquer des bouchages et rendent nécessaire un arrêt de l'atelier en vue de nettoyage.

Les inhibiteurs de polymérisation recommandés dans FR 2 592 040 pour stabiliser le milieu réactionnel sont la phénothiazine (PTZ), l'hydroquinone (HQ), le bleu de méthylène, le sulfate de fer, l'acétate ou le sulfate de cuivre.

La demande de brevet US 2002/0161260 portant sur un procédé de fabrication d'anhydrides d'acides carboxyliques insaturés par transanhydrification en présence d'un catalyseur préconise quant à elle l'utilisation d'inhibiteurs de polymérisation tels que l'hydroquinone (HQ), l'éther méthylique d'hydroquinone (EMHQ), la phénothiazine (PTZ), le 2,4-diméthyl 6-terbutyl phénol (TOPANOL^{®}A), le 2,6-diterbutyl 4-méthylphénol (TOPANOL^{®}O ou Baht), l'IRGANOX^{®} 1010 (commercialisé par CIBA AG Corporation), le N,N'-diphényl-p-phénylène diamine, ou leurs mélanges.

Dans EP 1 273 565, le TOPANOL^{®}A et le BHT, pris seuls ou en mélange sont choisis pour éviter le risque de polymérisation dans le réacteur et la colonne à distiller lors de la synthèse et la purification de l'anhydride (méth)acrylique.

Dans EP 1 319 650, 4-hydroxy-2,2,6,6-tetramethyl-piperidine-N-oxyl (HO-TEMPO) est choisi pour éviter le risque de polymérisation dans le réacteur et la colonne à distiller lors de la synthèse et la purification de l'anhydride (méth)acrylique.

Dans ces conditions de stabilisation habituelles, il est observé malgré tout, lors de la synthèse d'anhydride (méth)acrylique, la présence d'un fin dépôt de poudre blanche qui encrasse les parois, le fond, les contre-pâles et l'arbre d'agitation du réacteur. De plus, cette poudre blanche colmate les filtres placés en amont du bac de stockage du brut réactionnel. Ce problème de colmatage oblige à nettoyer régulièrement les filtres, ce qui n'est pas facile avec des produits aussi lacrymogènes que l'anhydride méthacrylique et surtout l'anhydride acrylique.

On connaît par ailleurs des inhibiteurs de polymérisation efficaces pour stabiliser les acides (méth)acryliques. On peut citer par exemple des composés N-oxyls en association avec un composé phénolique et la phénothiazine (EP 620 206), ou en association avec un sel de manganèse ou un sel de cuivre (EP 685 447) ou encore en association avec un dérivé phosphine ou un sel de cobalt (EP 810 196). Selon la demande GB 2 285 983, la polymérisation durant la distillation de composés vinyliques est inhibée à l'aide d'un composé dithiocarbamate de cuivre en présence d'un métal tel que le chrome, le manganèse, le titane ou le cobalt et en présence de phénothiazine, hydroquinone, p-méthoxyphénol, crésol, phénol, tert-butyl catéchol, diphénylamine ou bleu de méthylène.

Il n'est pas démontré que ces différents systèmes présentant une efficacité vis-à-vis des acides (méth)acryliques soient efficaces en présence d'anhydrides (méth)acryliques.

En effet, certains de ces stabilisants peuvent être estérifiés par les anhydrides et de ce fait, consommés dans le milieu réactionnel. Il a été montré que dans ces conditions ils perdent leur efficacité. C'est notamment le cas des composés tels que l'hydroquinone (HQ) ou l'éther méthylique d'hydroquinone (EMHQ).

La difficulté du choix d'un inhibiteur de polymérisation résulte du fait que les anhydrides (méth)acryliques et les acides (méth)acryliques ne sont pas sensibles aux mêmes inhibiteurs de polymérisation.

Lors de la réaction de transanhydrification entre l'acide (méth)acrylique et l'anhydride acétique, le milieu réactionnel contient les espèces suivantes :
- acide (méth)acrylique
- anhydride acétique
- anhydride (méth)acrylique
- anhydride mixte (méth)acrylique / acétique
- acide acétique

Il importe donc pour assurer une bonne stabilisation du milieu réactionnel, d'utiliser un inhibiteur de polymérisation ou un mélange d'inhibiteurs de polymérisation, capable de stabiliser l'ensemble des monomères polymérisables présents dans le milieu, plus particulièrement à la fois les anhydrides (méth)acryliques et mixtes, mais aussi les acides (méth)acryliques.

La société déposante a donc recherché un système d'inhibiteur(s) de polymérisation capable de stabiliser à la fois les anhydrides (méth)acryliques et les acides (méth)acryliques, pour résoudre les problèmes d'encrassement et de colmatage des filtres lors de la fabrication d'anhydride (méth)acrylique par transanhydrification.

La présente invention a donc pour objet un procédé amélioré de fabrication d'anhydride (méth)acrylique par transanhydrification entre l'acide (méth)acrylique et l'anhydride acétique en présence d'air et en présence d'au moins un inhibiteur de polymérisation, **caractérisé en ce que** l'inhibiteur de polymérisation est choisi dans le groupe constitué par (a) les sels métalliques de l'acide thiocarbamique ou dithiocarbamique et leurs mélanges avec un dérivé phénolique ou la phénothiazine et ses dérivés, et (b) les composés N-oxyls en mélange avec le 2,6-diterbutyl 4-méthyl phénol pris seul ou en présence de 2,4-diméthyl 6-terbutyl phénol.

Ces nouveaux systèmes stabilisants présentent de façon surprenante un effet remarquable et leur utilisation en présence d'air permet de supprimer le dépôt encrassant de poudre blanche tel que décrit précédemment, non seulement lors de la fabrication de l'anhydride (méth)acrylique, mais aussi durant sa purification, son stockage et son transport.

L'invention concerne également l'utilisation d'au moins un inhibiteur de polymérisation choisi dans le groupe constitué par (a) les sels métalliques de l'acide thiocarbamique ou dithiocarbamique et leurs mélanges avec un dérivé phénolique ou la phénothiazine et ses dérivés, et (b) les composés N-oxyls en mélange avec le 2,6-diterbutyl 4-méthyl phénol pris seul ou en présence de 2,6-diterbutyl 4-méthyl phénol et le 2,4-diméthyl 6-terbutyl phénol, pour la fabrication, la purification, le stockage ou le transport de l'anhydride (méth)acrylique.

Les sels métalliques de l'acide thiocarbamique ou dithiocarbamique sont choisis de préférence parmi les dialkyl dithiocarbamates de cuivre dans lesquels les groupes alkyls, identiques ou différents, linéaires ou ramifiés ont un nombre d'atomes de carbone allant de 1 à 8. On utilisera plus particulièrement le diéthyldithiocarbamate de cuivre ou le dibutyldithiocarbamate de cuivre.

Les dérivés phénoliques sont choisis parmi les dérivés phénoliques encombrés, c'est-à-dire substitués par des radicaux alkyle, identiques ou différents, linéaires ou ramifiés, ayant un nombre d'atomes de carbone allant de 1 à 8. On utilisera de préférence le 2,6-diterbutyl 4-méthyl phénol (BHT), le 2,4-diméthyl 6-terbutyl phénol (TOPANOL^{®}A).

La phénothiazine et ses dérivés tels que le bleu de méthylène peuvent être utilisés en association avec un sel métallique de l'acide thiocarbamique ou dithiocarbamique.

A titre d'illustration de composés N-oxyls utilisables selon l'invention, on peut citer les dérivés du 2,2,6,6-tétraméthyl-1-pipéridinyloxy (communément appelé TEMPO), notamment le 4-hydroxy-2,2,6,6-tétraméthyl-1-pipéridinyloxy (4-hydroxy TEMPO), le 4-méthoxy-2,2,6,6-tétraméthyl-1-pipéridinyloxy (4-méthoxy TEMPO), le 4-oxo-2,2,6,6-tétraméthyl-1-pipéridinyloxy (4-oxo TEMPO) ou le 4-amino-2,2,6,6-tétraméthyl-1-pipéridinyloxy (4-amino TEMPO).

Selon l'invention, les composés N-oxyls sont utilisés en mélange avec le 2,6-diterbutyl 4-méthyl phénol (BHT) pris seul ou en présence de 2,4-diméthyl 6-terbutyl phénol (TOPANOL^{®}A).

Selon un mode de réalisation particulier de l'invention, on utilise un mélange de 4-hydroxy TEMPO avec du BHT ou un mélange de 4-hydroxy TEMPO avec du BHT et du TOPANOL^{®}A.

La quantité d'inhibiteurs de polymérisation, pris seuls ou en mélanges, mise en oeuvre dans le réacteur est comprise entre 50 et 5000 ppm, plus particulièrement entre 300 et 4000 ppm et encore plus particulièrement entre 1000 et 3000 ppm par rapport à la masse totale des réactifs utilisés.

La réaction peut être mise en oeuvre dans un réacteur surmonté d'une colonne à distiller. On effectue alors de préférence une double stabilisation, en introduisant au moins un inhibiteur de polymérisation dans le réacteur et au moins un inhibiteur de polymérisation dans la colonne à distiller. Ainsi, on évite tout risque de polymérisation dans le réacteur et dans la colonne. L'inhibiteur du réacteur est de préférence introduit dans la charge initiale des réactifs. L'inhibiteur de la colonne à distiller est de préférence introduit dans la colonne à distiller, en tête de colonne dans le reflux, tout au long de la synthèse, en solution de concentration comprise entre 0,2 et 6% dans l'acide (méth)acrylique, l'anhydride acétique, l'acide acétique ou l'anhydride (méth)acrylique, particulièrement en solution à 5% dans de l'acide acétique. Le débit d'introduction de l'inhibiteur dans la colonne est ajusté de façon à avoir 2000 à 4000 ppm d'inhibiteur dans le produit final du réacteur.

En général, le réacteur est agité et chauffé par circulation de fluide caloporteur dans une double enveloppe ou par recirculation au travers d'un échangeur de chaleur extérieur. La colonne à distiller a de préférence une efficacité supérieure à 10 plateaux théoriques. Le garnissage de la colonne peut être un garnissage classique, en vrac ou structuré, ou un mélange de ces deux types de garnissage. La température de réaction est généralement comprise entre 50 et 120°C, de préférence entre 85 et 105°C. La pression est ajustée en fonction de la température de réaction choisie. En général, elle est comprise entre 20 et 200 mm de Hg. La réaction peut être effectuée en mode isobare, c'est-à-dire en fixant la pression et en faisant évoluer la température jusqu'à une valeur limite fixée de préférence entre 85 et 105°C, ou en mode isotherme, à savoir en fixant la température et en ajustant la pression dans l'installation tout au long de la réaction pour maintenir cette température. La température en tête de colonne est avantageusement ajustée durant la réaction, en fonction de la pression, de façon à correspondre à la température de distillation de l'acide acétique. En opérant ainsi, on obtient une fraction de tête contenant plus de 99% d'acide acétique.

Un bullage d'air est effectué durant toute la réaction.

Selon un mode de réalisation préféré de l'invention, l'acide acétique produit par la réaction est distillé au fur et à mesure de sa formation afin de déplacer les équilibres thermodynamiques. Selon un autre mode de réalisation préféré de l'invention, l'acide acétique éliminé est remplacé au moins partiellement par introduction dans le milieu réactionnel, au cours de la réaction, d'anhydride acétique et/ou d'acide (méth)acrylique.

Le brut obtenu peut subir une étape ultérieure de distillation, le cas échéant après étêtement, sur une colonne à distiller ou à l'aide d'un appareil à court temps de séjour tel qu'un évaporateur à film.

### Exemples

Les exemples suivants illustrent la présente invention sans toutefois en limiter la portée. Les pourcentages y sont exprimés en masses.

Les abréviations suivantes y sont utilisées :
AMA : acide (méth)acrylique
AA : acide acrylique
AMA₂O : anhydride méthacrylique
AA₂O : anhydride acrylique
Ac₂O : anhydride acétique
AcOH : acide acétique
Mixte : anhydride mixte acrylique/acétique ou anhydride méthacrylique/acétique selon le cas.
CB : dibutyl dithiocarbamate de cuivre
BHT : 2,6-diterbutyl 4-méthyl phénol
PTZ : phénothiazine
HQ : hydroquinone
EMHQ : éther méthylique d'hydroquinone
TOPANOL^{®}A : 2,4-diméthyl 6-terbutyl phénol
OHT : 4-hydroxy TEMPO : 2,2,6,6 tétraméthyl pipéridine N-oxyl.

### Exemples 1 à 9 :

Dans un réacteur agité mécaniquement (agitateur type ancre) en verre à double enveloppe alimentée par de l'huile à 125°C, surmonté par une colonne à distiller ayant un garnissage structuré Multiknit Sulzer, d'efficacité correspondant à 12 plateaux théoriques, avec condenseur, tête de reflux, séparateur à vide, recette et pièges, l'ensemble pouvant fonctionner sous vide, on introduit 255 g (2,5 moles) d'Ac₂O, 344,9 g (4,01 moles) d'AMA et un (ou des) inhibiteur(s) de polymérisation indiqué(s) dans le tableau 1.

Un bullage d'air (0,2 L/h) est maintenu dans le milieu réactionnel pendant toute la durée de la synthèse.

Pendant la phase réactionnelle, l'acide acétique produit par la réaction est distillé au fur et à mesure de sa formation afin de déplacer les équilibres thermodynamiques dans le sens de la formation de l'AMA₂O.

La température de réaction est maintenue à 93°C en faisant progressivement baisser la pression de service de 140 à 18 mm de Hg.

On récupère ainsi 230 ml d'une première fraction contenant 99% d'AcOH et 0,9% d'Ac₂O. L'excès d'Ac₂O, l'anhydride mixte et le complément d'AcOH formé sont ensuite éliminés par distillation sous 16 mm de Hg.

Le brut contenu dans le réacteur qui contient 95% de AMA₂O formé est ensuite refroidi à température ambiante et filtré. Il est stocké pour être éventuellement distillé afin d'obtenir un AMA₂O à plus de 99% de pureté. L'installation utilisée pour la synthèse du brut peut être utilisée pour effectuer la distillation.

L'examen de l'état de propreté du réacteur et de ses annexes renseigne sur l'efficacité des inhibiteurs mis en oeuvre.

Les concentrations en inhibiteurs dans le tableau 1 sont exprimées en parties par million (ppm) par rapport à la charge totale des réactifs introduits dans le réacteur. Les faibles concentrations en OHT permettent d'éviter des problèmes de coloration.

**Tableau 1**

| N° exemple | Inhibiteur | Conc, ppm | Observations |
|---|---|---|---|
| 1 | TOPANOL^{®}A | 1000 | Fort encrassement sur les parois et sur le dôme du réacteur. Brut trouble. Précipitation de solide, à froid après filtration dans le brut |
| comparatif | | | |
| 2 | BHT | 1000 | Même conclusion qu'à l'exemple 1 |
| comparatif | | | |
| 3 | TOPANOL^{®}A | 1000 | Même conclusion qu'à l'exemple 1 |
| comparatif | PTZ | 200 | |
| 4 | BHT | 1000 | Même conclusion qu'à l'exemple 1 |
| comparatif | PTZ | 200 | |
| 5 | TOPANOL^{®}A | 1000 | Encrassement des parois réduit. Précipitation de solide, à froid après filtration en moindre quantité qu'à l'exemple 1. |
| comparatif | BHT | 1000 | |
| | EMHQ | 8000 | |
| 6 | TOPANOL^{®}A | 1000 | Encrassement des: parois du réacteur. Précipitation de solides dans le brut froid après filtration. |
| comparatif | OHT | 200 | |
| 7 | BHT | 1000 | Réacteur parfaitement propre. Peu de précipité à froid, après filtration, dans le brut |
| | OHT | 200 | |
| 8 | BHT | 1000 | Réacteur parfaitement propre. Pas de précipité dans le brut froid après filtration. |
| | TOPANOL^{®}A | 1000 | |
| | OHT | 200 | |
| 9 | CB | 2000 | Réacteur parfaitement propre. Pas de précipité dans le brut froid après filtration mais brut légèrement coloré |

### Exemples 10 à 13 :

Dans l'installation décrite précédemment, on charge 408g d'Ac₂O (4 moles), 403,2 g d'AA (5,6 moles) et un (ou des) inhibiteur(s) de polymérisation indiqué(s) dans le tableau 2.

La température de réaction est maintenue aux environs de 90°C en baissant progressivement la pression de service dans l'installation de 160 à 18 mm de Hg.

Un bullage d'air (0,2 L/h) est maintenu dans le milieu réactionnel pendant toute la durée de la synthèse.

Pendant la phase réactionnelle, l'acide acétique produit par la réaction est distillé au fur et à mesure de sa formation afin de déplacer les équilibres thermodynamiques dans le sens de la formation de l'AA₂O.

Après élimination de l'excès d'Ac₂O par distillation sous vide, l'AA₂O pur est distillé sous 18 mm de Hg.

L'examen de l'état de propreté du réacteur et de ses annexes après distillation de l'AA₂O renseigne sur l'efficacité des inhibiteurs mis en oeuvre.

Les concentrations en inhibiteurs sont exprimées en parties par million (ppm) par rapport à la charge totale des réactifs introduits dans le réacteur.

**Tableau 2**

| N° exemple | Inhibiteur | Conc, ppm | Observations |
|---|---|---|---|
| 10 | CB | 2000 | Réacteur propre après distillation de l'AA₂O. Résidu fluide |
| 11 | CB | 250 | Même conclusion qu'à |
| | PTZ | 1000 | l'exemple 10 |
| 12 | CB | 500 | Même conclusion qu'à |
| | BHT | 1000 | l'exemple 10 |
| 13 | TOPANOL^{®}A | 1000 | |
| | BHT | 1000 | Faible encrassement des parois |
| | OHT | 200 | du réacteur |

## Revendications

1. Procédé de fabrication d'anhydrides (méth)acryliques par transanhydrification entre l'acide (méth)acrylique et l'anhydride acétique, en présence d'air et en présence d'au moins un inhibiteur de polymérisation, **caractérisé en ce que** l'inhibiteur de polymérisation est choisi dans le groupe constitué par (a) les sels métalliques de l'acide thiocarbamique ou dithiocarbamique et leurs mélanges avec un dérivé phénolique ou la phénothiazine et ses dérivés, et (b) les composés N-oxyls en mélange avec le 2,6-diterbutyl 4-méthyl phénol pris seul ou en présence de 2,4-diméthyl 6-terbutyl phénol.

2. Procédé selon la revendication 1, **caractérisé en ce que** le sel métallique de l'acide dithiocarbamique est choisi parmi les dialkyl dithiocarbamates de cuivre dans lesquels les groupes alkyls, identiques ou différents, linéaires ou ramifiés ont un nombre d'atomes de carbone allant de 1 à 8.

3. Procédé selon la revendication 1 ou 2, **caractérisé en ce que** l'on utilise le diéthyldithiocarbamate de cuivre ou le dibutyldithiocarbamate de cuivre.

4. Procédé selon l'une des revendications 1 à 3, **caractérisé en ce que** le dérivé phénolique est le 2,6-diterbutyl 4-méthyl phénol ou le 2,4-diméthyl 6-terbutyl phénol.

5. Procédé selon la revendication 1, **caractérisé en ce que** le composé N-oxyl est un dérivé du 2,2,6,6-tétraméthyl-1-pipéridinyloxy (TEMPO).

6. Procédé selon la revendication 5, **caractérisé en ce que** le composé N-oxyl est le 4-hydroxy TEMPO, le 4-méthoxy TEMPO, le 4-oxo TEMPO ou le 4-amino TEMPO.

7. Procédé selon la revendication 5 ou 6, **caractérisé en ce que** le composé N-oxyl est le 4-hydroxy TEMPO.

8. Procédé selon l'une des revendications 1 à 7, **caractérisé en ce que** la quantité d'inhibiteurs, pris seuls ou en mélanges, mise en oeuvre dans le réacteur est comprise entre 50 et 5000 ppm, plus particulièrement entre 300 et 4000 ppm et encore plus particulièrement entre 1000 et 3000 ppm par rapport à la masse totale des réactifs utilisés.

9. Procédé selon l'une des revendications 1 à 8, **caractérisé en ce que** la réaction est mise en oeuvre dans un réacteur surmonté d'une colonne à distiller.

10. Procédé selon la revendication 9, **caractérisé en ce que** l'on introduit au moins un inhibiteur de polymérisation dans le réacteur et au moins un inhibiteur de polymérisation dans la colonne à distiller.

11. Procédé selon la revendication 10 **caractérisé en ce que** l'inhibiteur de la colonne à distiller est introduit dans la colonne à distiller tout au long de la synthèse, en solution de concentration comprise entre 0,2 et 6% dans l'acide (méth)acrylique, l'anhydride acétique, l'acide acétique ou l'anhydride (méth)acrylique.

12. Utilisation d'au moins un inhibiteur de polymérisation choisi dans le groupe constitué par (a) les sels métalliques de l'acide thiocarbamique ou dithiocarbamique, et leurs mélanges avec un dérivé phénolique ou avec la phénothiazine et ses dérivés, et (b) les composés N-oxyls en mélange avec le 2,6-diterbutyl 4-méthyl phénol pris seul ou en présence de 2,6-diterbutyl 4-méthyl phénol et le 2,4-diméthyl 6-terbutyl phénol, pour la fabrication, la purification, le stockage ou le transport de l'anhydride (méth)acrylique.

## Claims

1. Method for the production of (meth)acrylic anhydrides by transanhydrification between (meth)-acrylic acid and acetic anhydride, in the presence of air and in the presence of at least one polymerization inhibitor, **characterized in that** the polymerization inhibitor is chosen from the group consisting of (a) metal salts of thiocarbamic acid or dithiocarbamic acid and mixtures thereof with a phenol derivative or phenothiazine and the derivatives thereof, and (b) N-oxyl compounds mixed with 2,6-di-tert-butyl-4-methylphenol taken on its own or in the presence of 2,4-dimethyl-6-tert-butylphenol.

2. Method according to Claim 1, **characterized in that** the metal salt of dithiocarbamic acid is chosen from copper dialkyl dithiocarbamates in which the linear or branched alkyl groups, which may be identical or different, contain a number of carbon atoms ranging from 1 to 8.

3. Method according to Claim 1 or 2, **characterized in that** copper diethyldithiocarbamate or copper dibutyldithiocarbamate is used.

4. Method according to one of Claims 1 to 3, **characterized in that** the phenol derivative is 2,6-di-tert-butyl-4-methylphenol or 2,4-dimethyl-6-tert-butylphenol.

5. Method according to Claim 1, **characterized in that** the N-oxyl compound is a 2,2,6,6-tetramethyl-1-piperidinyloxy (TEMPO) derivative.

6. Method according to Claim 5, **characterized in that** the N-oxyl compound is 4-hydroxy TEMPO, 4-methoxy TEMPO, 4-oxo TEMPO or 4-amino TEMPO.

7. Method according to Claim 5 or 6, **characterized in that** the N-oxyl compound is 4-hydroxy TEMPO.

8. Method according to one of Claims 1 to 7, **characterized in that** the amount of inhibitors, taken on their own or as mixtures, used in the reactor is between 50 and 5000 ppm, more particularly between 300 and 4000 ppm, and even more particularly between 1000 and 3000 ppm, relative to the total mass of the reactants used.

9. Method according to one of Claims 1 to 8, **characterized in that** the reaction is carried out in a reactor surmounted by a distillation column.

10. Method according to Claim 9, **characterized in that** at least one polymerization inhibitor is introduced into the reactor and at least one polymerization inhibitor is introduced into the distillation column.

11. Method according to Claim 10, **characterized in that** the inhibitor of the distillation column is introduced into the distillation column throughout the synthesis, in solution at a concentration of between 0.2% and 6% in (meth)acrylic acid, acetic anhydride, acetic acid or (meth)acrylic anhydride.

12. Use of at least one polymerization inhibitor chosen from the group consisting of (a) metal salts of thiocarbamic acid or dithiocarbamic acid and mixtures thereof with a phenol derivative or with phenothiazine and derivatives thereof, and (b) N-oxyl compounds mixed with 2,6-di-tert-butyl-4-methylphenol taken on its own or in the presence of 2,6-di-tert-butyl-4-methylphenol and 2,4-dimethyl-6-tert-butylphenol, for the production, purification, storage or transport of (meth)acrylic anhydride.

## Patentansprüche

1. Verfahren zur Herstellung von (Meth)acrylsäureanhydrid durch Umanhydridisierung zwischen (Meth)-acrylsäure und Essigsäureanhydrid in Gegenwart von Luft und in Gegenwart von mindestens einem Polymerisationsinhibitor, **dadurch gekennzeichnet, daß** man den Polymerisationsinhibitor aus der Gruppe bestehend aus (a) Metallsalzen von Thiocarbamidsäure oder Dithiocarbamidsäure und Mischungen davon mit einem Phenolderivat oder Phenothiazin und Derivaten davon und (b) N-Oxylverbindungen in Abmischung mit 2,6-Di-tert-butyl-4-methylphenol für sich alleine oder in Gegenwart von 2,4-Dimethyl-6-tert-butylphenol auswählt.

2. Verfahren nach Anspruch 1, **dadurch gekennzeichnet, daß** man das Metallsalz von Dithiocarbamidsäure unter Kupferdialkyldithiocarbamaten, worin die Alkylgruppen gleich oder verschieden und linear oder verzweigt sind und 1 bis 8 Kohlenstoffatome aufweisen, auswählt.

3. Verfahren nach Anspruch 1 oder 2, **dadurch gekennzeichnet, daß** man Kupferdiethyldithiocarbamat oder Kupferdibutyldithiocarbamat verwendet.

4. Verfahren nach einem der Ansprüche 1 bis 3, **dadurch gekennzeichnet, daß** es sich bei dem Phenolderivat um 2,6-Di-tert-butyl-4-methylphenol oder 2,4-Dimethyl-6-tert-butylphenol handelt.

5. Verfahren nach Anspruch 1, **dadurch gekennzeichnet, daß** es sich bei der N-Oxylverbindung um ein Derivat von 2,2,6,6-Tetramethyl-1-piperidinyloxy (TEMPO) handelt.

6. Verfahren nach Anspruch 5, **dadurch gekennzeichnet, daß** es sich bei der N-Oxylverbindung um 4-Hydroxy-TEMPO, 4-Methoxy-TEMPO, 4-Oxo-TEMPO oder 4-Amino-TEMPO handelt.

7. Verfahren nach Anspruch 5 oder 6, **dadurch gekennzeichnet, daß** es sich bei der N-Oxylverbindung um 4-Hydroxy-TEMPO handelt.

8. Verfahren nach einem der Ansprüche 1 bis 7, **dadurch gekennzeichnet, daß** die im Reaktor verwendete Menge an Inhibitoren, für sich alleine oder in Abmischungen, zwischen 50 und 5000 ppm, speziell zwischen 300 und 4000 ppm und noch spezieller zwischen 1000 und 3000 ppm, bezogen auf die Gesamtmasse der verwendeten Reaktanten, liegt.

9. Verfahren nach einem der Ansprüche 1 bis 8, **dadurch gekennzeichnet, daß** man die Umsetzung in einem Reaktor mit aufgesetzter Destillationskolonne durchführt.

10. Verfahren nach Anspruch 9, **dadurch gekennzeichnet, daß** man mindestens einen Polymerisationsinhibitor in den Reaktor und mindestens einen Polymerisationsinhibitor in die Destillationskolonne einträgt.

11. Verfahren nach Anspruch 10, **dadurch gekennzeichnet, daß** man den Inhibitor für die Destillationskolonne über die gesamte Synthese hinweg in Lösung mit einer Konzentration zwischen 0,2 und 6% in (Meth)acrylsäure, Essigsäureanhydrid, Essigsäure oder (Meth)acrylsäureanhydrid in die Destillationskolonne einträgt.

12. Verwendung mindestens eines aus der Gruppe bestehend aus (a) Metallsalzen von Thiocarbamidsäure oder Dithiocarbamidsäure und Mischungen davon mit einem Phenolderivat oder Phenothiazin und Derivaten davon und (b) N-Oxylverbindungen in Abmischung mit 2,6-Di-tert-butyl-4-methylphenol für sich alleine oder in Gegenwart von 2,6-Di-tert-butyl-4-methylphenol und 2,4-Dimethyl-6-tert-butylphenol ausgewählten Polymerisationsinhibitors zur Herstellung, zur Reinigung, zur Lagerung oder zum Transport von (Meth)acrylsäureanhydrid.
